# EUROPÄISCHE PATENTSCHRIFT

(11) **EP 4 129 247 B1**
(45) Veröffentlichungstag und Bekanntmachung des Hinweises auf die Patenterteilung: **29.10.2025**
(21) Anmeldenummer: 22000191.1
(22) Anmeldetag: 02.08.2022
(51) Int. Cl.: A61F 5/02

(54) **SYSTEM ZUR KORREKTUR EINER KÖRPERHALTUNG**
BODY POSTURE CORRECTING SYSTEM
SYSTÈME DE CORRECTION D'UNE POSTURE

(30) Priorität: 07.08.2021 DE 102021004118
(43) Veröffentlichungstag der Anmeldung: 08.02.2023
(73) Patentinhaber: Baierle, Tobias, 69115 Heidelberg (DE)
(72) Erfinder: Baierle, Tobias, 69115 Heidelberg (DE)

(56) Entgegenhaltungen:
- CN-U- 210 044 203
- US-A1- 2012 197 160
- US-A1- 2020 268 318

## Beschreibung

Die Erfindung betrifft ein System zur Korrektur einer Körperhaltung.

Schlechte Körperhaltung ist häufig die Ursache einer Vielzahl von Beschwerden. Zum Beispiel ist der Auslöser vieler Schulter- und Wirbelsäulenpathologien eine Fehlstellung der Schulter und/oder Wirbelsäule. Wird diese Fehlstellung korrigiert, führt dies zur Verbesserung von muskulären Verspannungen und Schmerzen.

Hierfür werden häufig sogenannte Haltungstrainer oder Gradehalter eingesetzt. Die Haltungstrainer/Gradehalter arbeiten nach zwei unterschiedlichen Prinzipien:
A) Durch Gurte mit fixierender oder korrigierender Schulterblatt-/ Wirbelsäulenfunktion. Diese schränken jedoch die Beweglichkeit stark ein und sind meist unangenehm zu tragen. Ein solches System offenbart beispielsweise die EP 2 490 631 B1. Diese Systeme sind in der Regel rein passiv, das heißt ohne Messung des Istzustandes und stützen ununterbrochen. Langfristig übernimmt der Gurt die stützende Funktion und kann somit sogar kontraproduktiv wirken.
B) Mit Neigungssensoren (häufig Beschleunigungssensoren oder Drehratensensoren) ausgestattete Haltungstrainer, die bei Körpervorbeugung einen Vibrationsimpuls auslösen (Erfassung des Istzustandes). Es gibt trägerloser Haltungstrainer oder fürs Reiten entwickelte Sensoren zur Kontrolle der aufrechten Kopfhaltung. Das Problem hierbei ist jedoch, dass viele Alltags- oder Sportaktivitäten in vorgeneigter Position stattfinden, z.B. bei der Hausarbeit und beim Golfen. Diese Art der Haltungstrainer ist hierfür gar nicht oder nur eingeschränkt verwendbar. Weiter ist es für die meisten Menschen unmöglich sich den ganzen Tag aufrecht zu halten, ohne durch die Anstrengung muskuläre Verspannungen zu bekommen, was damit dem eigentlichen Ziel entgegenwirkt.

Ein solches System ist aus der WO 2019/162170 A1 für Reiter bekannt, das ein taktiles Feedbacksystem beinhaltet.

Die EP 2 877 091 B1 offenbart eine Einlegesohle für einen Schuh, mittels derer eine mehraxiale Spannungsanalyse des Schuhträgers durchgeführt werden kann.

Die WO 2011/032575 A1 offenbart ein System zum Erfassen von Funktionsparametern zur Charakterisierung von Bewegungsabläufen am menschlichen Körper, aufweisend einen Biegesensor mit mindestens einem Dehnungsmessstreifen.

Die CN 210044203 U beschreibt einen intelligenten Gurt, der die Sitzhaltung überwacht und korrigiert.

Die US 2012/197160 A1 beschreibt eine Vorrichtung zur Erfassung und/oder Beeinflussung der Körperhaltung mit einem flexiblen Trägerelement, an dem eine Fixiereinrichtung zur Anordnung der Vorrichtung in oder unterhalb der Taillenhöhe eines Trägers der Vorrichtung angeordnet ist.

Der Erfindung liegt die Aufgabe zugrunde, ein System vorzuschlagen, das Haltungsfehler erkennt, die Bewegung nicht einschränkt, angenehm zu tragen ist, und auch in allen Alltagsaktivitäten zu verwenden ist.

Die Aufgabe wird gelöst durch ein System, umfassend: eine Trägereinrichtung zur Anbringung am menschlichen Körper mit zumindest einem ersten Dehnungsbereich, wobei der erste Dehnungsbereich einen Formgedächtniswerkstoff mit einem ersten Ende und einem zweiten Ende beinhaltet, insbesondere zumindest einen Faden aus einem Formgedächtniswerkstoff oder eine Flachformstruktur beinhaltet, und wobei sich bei einer Relativbewegung eines ersten Körperteils zu einem zweiten Körperteil eine, insbesondere positive, Dehnung des ersten Dehnungsbereichs mit dem Formgedächtniswerkstoff ergibt, wobei es sich bei dem ersten und zweiten Körperteil um eine Extremität, Gelenk, Wirbel, Muskel oder einen Knochen handelt; zumindest einen Sensor, der mit dem ersten und zweiten Ende verbunden ist, und dazu ausgestaltet ist, eine Dehnung zu detektieren und damit korrespondierende Signale an eine Datenverarbeitungseinheit zu senden; die Datenverarbeitungseinheit, die mit dem Sensor verbunden ist; und zumindest eine Feedbackeinheit, die mit der Datenverarbeitungseinheit verbunden ist, wobei die Datenverarbeitungseinheit dazu ausgestaltet ist, ein Signal an die Feedbackeinheit zu senden, wenn die Dehnung größer als ein Schwellenwert ist und diese eine Rückmeldung an den Körper abgibt.

Die Messung erfolgt in einer Ausgestaltung kontinuierlich und der Schwellwert kann beliebig gesetzt werden. In anderen Worten: die Hardware (Sensor) misst kontinuierlich über den gesamten Weg, während die Software (in der Datenverarbeitungseinheit) als Schalter funktioniert und ab einem bestimmten Schwellwert einen Alarm auslöst und eine Rückmeldung an den Körper abgibt.

In einer Ausgestaltung wird der Schwellwert dynamisch, gegebenenfalls mit Hysterese, gesetzt. Hysterese beschreibt Verhalten, bei dem die Ausgangsgröße nicht allein von der unabhängig veränderlichen Eingangsgröße, sondern auch vom vorherigen Zustand der Ausgangsgröße abhängt. Das System kann also - abhängig von der Vorgeschichte - bei gleicher Eingangsgröße einen von mehreren möglichen Zuständen einnehmen. Das Feedback wird also in einer Ausgestaltung nicht immer bei einem bestimmten Wert ausgelöst, sondern abhängig von der Vorgeschichte, etwa wann zuletzt ein Feedback ausgelöst wurde oder wie weit der Schwellenwert überschritten wurde.

Im Gegensatz zu den Haltungshilfen aus dem Stand der Technik, wird mit der vorliegenden Idee eine Schulterblattstabilisation und Haltungskorrektur der Wirbelsäule erreicht. Die Bewegung ist nicht einschränkt, das System ist angenehm zu tragen, besonders auch in vorgebeugter Haltung, also während allen Alltagsaktivitäten, die in Körperverneigung ausgeführt werden, und beim Sport. Es wird eine aktive Schulter- und Haltungskorrektur gefördert. Dies wird dadurch erreicht, dass die eigentliche Verformung des Körperabschnitts - und die damit einhergehende Längen- bzw. Winkeländerung - vermessen wird.

Es geht also grundlegend darum, die Wirbelsäule, hier insbesondere die Halswirbelsäule und die Brustwirbelsäule, vor allem auch bei Bewegung gerade zu halten bzw. wenn dies nicht (mehr) der Fall ist, als Nutzer entsprechendes Feedback zu bekommen.

In einer Ausgestaltung wird das System durch eine Software-Steuerung individuell eingestellt. Die aktive Schulter- und Haltungskorrektur kann mit diesem Produkt alltagstauglich angewendet werden und über die Software-Steuerung an die individuellen Bedürfnisse des Trägers angepasst werden. Die Software-Steuerung erfolgt in einer Ausgestaltung über eine App (siehe unten).

Da die Trägereinrichtung im Gegensatz zum Stand der Technik keine fixierende oder passiv korrigierende Funktion erfüllen muss, kann das beanspruchte System ohne eine bewegungseinschränkende Fixierung eine Haltungskorrektur erzielen, es schneidet praktisch nicht ein und lässt die volle Bewegungsfreiheit zu.

Die Erfindung kann auch, im Gegensatz zu den mit Neigungssensoren ausgestatteten Haltungsstabilisatoren, die aufrechte Haltung und Schulterblattaufrichtung und -stabilisation aktivieren, besonders auch in vorgebeugter Haltung, also während allen Alltagsaktivitäten, die in Körperverneigung ausgeführt werden.

Die Fehlstellung der Schulter/Wirbelsäule, wird durch das Biofeedback System aktiv korrigiert, die aufrichtende Muskulatur gestärkt und somit kommt es zu einer physiologischen Haltungsverbesserung.

Eine Ausgestaltung sieht vor, dass der Sensor zur Messung des Widerstands ausgestaltet ist, insbesondere umfasst der Sensor eine Stromquelle, insbesondere eine Konstantstromquelle, und eine Spannungsmesseinheit. In einer Ausgestaltung wird dazu ein entsprechender ASIC (engl. application-specific integrated circuit, anwendungsspezifische integrierte Schaltung) verwendet. Es sind aber auch andere Messverfahren zum Auslesen der Widerstandsänderung denkbar.

Eine Ausgestaltung sieht vor, dass der Formgedächtniswerkstoff, insbesondere der zumindest eine Faden oder die eine Flachformstruktur, in den ersten Dehnungsbereich eingewebt, gestrickt, gewirkt oder getuftet ist.

Eine Ausgestaltung sieht vor, dass es sich bei dem Formgedächtniswerkstoff um eine Nickel-Titan-Basislegierung handelt.

Eine Ausgestaltung sieht vor, dass das System umfasst: einen Bluetooth-Chip, der mit der Datenverarbeitungseinheit verbunden ist, und dazu ausgestaltet ist eine Verbindung mit einem Mobilgerät aufzubauen. Auf dem Mobilgerät befindet sich eine entsprechende App, mittels welcher die Software-Steuerung und Einstellungen durchgeführt werden. In einer Ausgestaltung kann das System auch über WLAN (Wifi), ANT, ANT+, Zigbee oder ähnlich Drahtlosprotokolle mit dem Mobilgerät verbunden werden. In einer Ausgestaltung umfasst das System einen USB-Anschluss über den ein externes Gerät, etwa ein Mobilgerät oder ein Computer angeschlossen werden, wobei dann darüber die Software-Steuerung erfolgt. Neben den Einstellungen können auch Messwerte ausgelesen und verändert werden. Die Berechnungen können auf der eingebauten Logik und/oder auf dem Mobilgerät stattfinden. Bei dem Mobilgerät handelt es sich etwa um ein Smartphone. Mit dem Mobilgerät, etwa dem Smartphone, können Smart Devices gekoppelt sein, etwa Smartwatches oder Smart Glasses. Auch kann die Smartwatch direkt mit dem System bzw. dem Funkchip (siehe oben), etwa dem Bluetooth-Chip, verbunden sein.

Eine Ausgestaltung sieht vor, dass das System einen zweiten Dehnungsbereich umfasst, der weicher ist als der erste Dehnungsbereich und sich beim Anziehen des Systems dehnt.

Eine Ausgestaltung sieht vor, dass das System einen dritten Dehnungsbereich umfasst, der härter ist als der erste Dehnungsbereich und eine Überlast des ersten Dehnungsbereichs verhindert.

In einer Ausgestaltung handelt es sich bei der Feedbackeinheit um einen Vibrationsmotor, einen Unwuchtmotor oder einen anderen Vibrationsgeber. In einer Ausgestaltung handelt es sich bei der Feedbackeinheit um eine Einheit zum Aussenden von Reizstrom, auf Englisch "electrical muscle stimulation" (EMS). In einer Ausgestaltung kann wird optisches oder akustisches Signal ausgegeben. Das System umfasst dann dazu entsprechende Gebereinheiten, etwa eine Leuchtdiode bzw. einen Lautsprecher. In einer Ausgestaltung wird über den Funkchip (siehe oben) ein entsprechendes Signal an das Mobilgerät gesendet und darauf eine Nachricht ausgegeben. Das Signal kann über das Mobilgerät auch an gekoppelte andere Smart Devices, etwa die Smartwatch, weitergeleitet werden. Der Nutzer bekommt dann das Feedback auf die Smartwatch. Auch kann die Smartwatch direkt mit dem System bzw. dem Funkchip verbunden sein.

Eine Ausgestaltung sieht vor, dass es sich bei dem ersten Körperteil um das Schulterblatt verbunden mit dem Schlüsselbein und beim zweiten Körperteil, um den Brustkorb, insbesondere um den hinteren Teil des Brustkorbs, handelt.

Im Detail erfolgt dies durch in-situ Messung der Schulterposition, indem der relative Abstand zwischen einem Brustwirbel, etwa Brustwirbel 7 (TH7), und Schlüsselbeingelenk ("AC Gelenk") bestimmt bzw. gemessen wird. Die in der WO 2019/162170 A1 beschriebene Lösung erfasst im Gegensatz hierzu Positions- oder Neigungsveränderungen eines Messpunktes der am zum messenden Objekt (Träger/Mensch) angebracht ist. Hierdurch können Fehlinterpretationen durch Lageveränderungen des Sensors auftreten, die nicht durch eine Veränderung der Schulterposition erfolgt sind. Der relative Abstand zwischen der Brustwirbelsäule und AC Gelenk wird daher direkt mechanisch, durch die Änderung einer Kraft oder eines Weges mittels Sensor, gemessen. Die gemessenen Sensordaten werden kontaktlos an eine Recheneinheit übertragen und können verarbeitet werden.

Das der Idee zugrunde liegende Prinzip lässt sich auch auf Relativbewegungen anderer Körperteile zueinander übertragen, wie beispielsweise das Sprunggelenk. Hier würde etwa bei einer Fußheberschwäche der Sensor die Möglichkeit bieten, dass wenn der Fuß durch fehlende neurale Ansteuerung beim Gehen in pathologische Plantarflexion (Fallfuß) geht, statt eines Signals der Feedbackeinheit (taktiles Biofeedback), eine elektrische Stimulation z.B. EMS-Strom zur Stimulation der perinodale Muskulatur (Fußhebemuskulatur) durch die Feedbackeinheit ausgelöst wird und so den Fallfuß verhindert. In einer Ausgestaltung wird dabei über die Ferse gemessen.

### Weitere Beispiele sind die folgenden:

Lendenwirbelsäule und Brustwirbelsäule: hier wird mit dem Sensor, der in diesem Bereich fixiert wird, das vorzeitige Bewegen in Kyphose (Vorneigung der Lendenwirbelsäule) gemessen und diesem über das Biofeedback beim Heben oder dem Zusammensinken beim Sitzen entgegengewirkt.

Knie: Der Sensor wird an einer Orthese/Bandage oder mit zwei Klebepads über und unterhalb an der Knieinnenseite fixiert, und das Feedback ausgelöst, wenn das Knie in X-Beinstellung geht (medialisiert) um damit die Beinachse aktiv zu korrigieren.

Fußgewölbe / Sprunggelenk: Die Feedbackeinheit wird an einer Orthese/Bandage oder zwei über einen mit Klebepads fixierten Gurt angebracht und ausgelöst, wenn das Fußgewölbe absinkt oder sich das Sprunggelenk in Hypersupination oder Hyperpronation bewegt, um die Achse oder das Gewölbe aktiv zu korrigieren und stabilisieren. Ebenfalls möglich ist eine elektrische Stimulation z.B. EMS-Strom zur Stimulation der stabilisierenden Muskulatur, des Fußgewölbes oder des Sprunggelenks.

Hüfte: Es werden zwei Klebepads über und unter dem Trochanter major fixiert, wobei dabei über den damit verbundenen Sensor und Feedbackeinheit das Absinken (Trendelburg) der gegenüberliegenden Hüfte mit Biofeedback (etwa der Abduktoren/Außenrotatoren) oder elektrischer Stimulation (EMS-Strom) verhindert wird.

Das grundlegende Prinzip ist auf alle sich voneinander wegbewegenden Körperabschnitte übertragbar und so für eine Vielzahl von Pathologien, bei denen eine aktive Korrektur durch Muskelaktivität gewünscht ist, einsetzbar. Hierbei kann entweder ein Biofeedback oder elektrischer Stimulation z.B. EMS-Strom zur Stimulation eingesetzt werden

In einer Ausgestaltung wird die Trägereinrichtung in verschiedenen Größen angeboten oder ist größenverstellbar, etwa durch Klettverschluss.

In einer Ausgestaltung ist zumindest einer der Komponenten Sensor, Feedbackeinheit und Datenverarbeitungseinheit abnehmbar ausgestaltet. Dann kann die Trägereinrichtung gewaschen werden, ohne die elektrischen Komponenten zu beschädigen. Bevorzugt sind alle Komponenten Sensor, Feedbackeinheit und Datenverarbeitungseinheit von der Trägereinrichtung abnehmbar.

Zusammengefasst offenbart die vorliegende Idee einen Haltungstrainer mit Biofeedback, das über einen Sensor die Distanzveränderung zusammenhängender Gelenkpartner messen und durch Biofeedback an den Träger eine aktive Schulterblattaufrichtung und Haltungskorrektur der Wirbelsäule gewährleistet. Dabei kann das System durch eine temporäre Funk-Verbindung, beispielsweise Bluetooth, Wifi oder proprietäre Funkprotokolle, mit einer Software/App auf einem Mobilgerät oder PC verbunden werden, Messwerte ausgelesen und Einstellungen verändert werden.

Dies wird anhand der nachfolgenden Figuren näherer erläutert.
- Fig. 1: zeigt das beanspruchte System.
- Fig. 2: zeigt das getragene System.
- Fig. 3a-d: zeigen Situationen des Trägers des Systems.
- Fig. 4a/b: zeigen die Dehnungsbereiche.
- Fig. 5: zeigt die Dehnungsbereiche am System.
- Fig. 6: zeigt eine Ausgestaltung eines Gehäuses.
- Fig. 7: zeigt eine Ausgestaltung eines Gehäuses.

In den Figuren sind gleiche Merkmale mit gleichen Bezugszeichen gekennzeichnet.

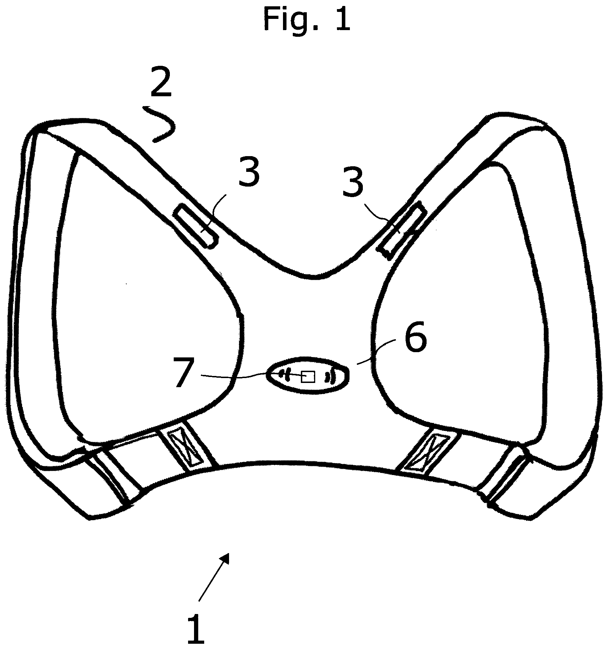

Das beanspruchte System zur Korrektur einer Körperhaltung in seiner Gesamtheit hat das Bezugszeichen 1 und ist in Fig. 1 dargestellt.

Das System 1 umfasst eine Trägereinrichtung 2 zur Anbringung desselben am menschlichen Körper 10. Die Trägereinrichtung 2 ist beispielsweise als Weste ausgebildet. In einer Ausgestaltung ist die Trägereinrichtung 2 als Büstenhalter ausgebildet. In einer Ausgestaltung ist die Trägereinrichtung 2 als eine Art Rucksack ausgebildet. In einer Ausgestaltung ist die Trägereinrichtung 2 als T-Shirt, Longsleeve, Unterhemd, Trikot, o.ä. ausgestaltet.

Die Trägereinrichtung 2 kann aus einem textilen Material bestehen, das beispielsweise innenseitig mit einer rutschfesten oder rutschhemmenden Beschichtung versehen ist, um die Trägereinrichtung 2 sicher an dem Körper 10 des Trägers des System 1 zu halten. Die Trägereinrichtung 2 kann neben einer Flexibilität auch eine Elastizität aufweisen, um ein möglichst enges Anliegen an dem Körper zu ermöglichen. Die Trägereinrichtung 2 kann im vorderen Bereich einen Reißverschluss aufweisen, über den die Trägereinrichtung 2 nach dem Anlegen geschlossen werden kann, um eine verbesserte Passform und einen besseren Sitz am Körper 10 zu bewirken. Statt eines Reißverschlusses können andere Verschlusseinrichtungen vorgesehen sein, beispielsweise ein Klettverschluss, Knöpfe oder andere Verbindungs- oder Fixiereinrichtungen.

In einer Ausgestaltung umfasst die Trägereinrichtung 2 über beide Schultern führende Träger zur vertikalen Stabilisierung und einem am Rücken oder vorn verschließbaren Band zur horizontalen und eigentlichen Stabilisierung. Es ist ein rückwärtiger Verschluss oder vorderseitiger Verschluss vorgesehen. Sowohl die beiden Schulterträger als auch das Rückenteil lassen sich zur Feinanpassung verstellen, z. B. ist der Rückenverschluss zur Weitenregulierung etwa in zwei oder drei verschieden weit entfernte Ösen einhakbar. Schulterträger und Rückenband sind so ausgestaltet, dass diese beim Tragen nicht in die Haut einschneiden.

Im Falle der Ausgestaltung der Trägereinrichtung 2 als T-Shirt, Longsleeve, Unterhemd, Trikot, o.ä. ist dieses eher enganliegend ausgestaltet. Die Trägereinrichtung 2 kann als "Smart Fabrics" oder "Smart Cloth" ausgestaltet sein. Dabei ist der Dehnungsbereich 3 und gegebenenfalls auch die Datenverarbeitungseinheit 6 (siehe unten) Teil der Trägereinrichtung 2.

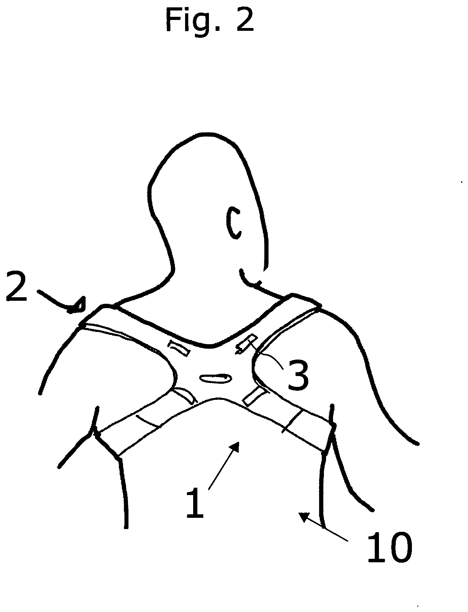

Fig. 2 zeigt das System 1 am Körper 10 eines Trägers.

Die Trägereinrichtung 2 umfasst zumindest einen ersten Dehnungsbereich 3. Zwar ist die Trägereinrichtung 2 wie oben erwähnt flexibel und elastisch, der erste Dehnungsbereich 3 ist aber vom restlichen Teil der Trägereinrichtung 2 mit seinen grundlegenden Eigenschaften des (textilen) Materials der Trägereinrichtung 2 zu unterscheiden. Der erste Dehnungsbereich 3 dehnt sich nur bei entsprechender Bewegung des Trägers (worauf im Folgenden eingegangen wird), während die Flexibilität/Elastizität der Trägereinrichtung 2 für ein angenehmes und komfortables Tragegefühl sorgt.

Der erste Dehnungsbereich 3 beinhaltet einen Formgedächtniswerkstoff mit einem ersten Ende 3a und einem zweiten Ende 3b, insbesondere zumindest einen Faden oder eine, geometrisch strukturierte, Flachformstruktur aus einem Formgedächtniswerkstoff. Formgedächtnislegierungen treten wie andere metallische Legierungen temperaturabhängig in unterschiedlichen Kristallsystemen auf. Zusätzlich kann bei Formgedächtnislegierungen das vorliegende Kristallsysteme durch eine äußere mechanische Spannung verändert werden (ohne Temperaturveränderung). Es gibt drei Arten des Formgedächtniseffektes (Einweg-Effekt, Zweiweg-Effekt und pseudoelastisches Verhalten) von denen sich bei der vorliegenden Anmeldung der pseudoelastische Effekt bestens eignet. Bei diesem Effekt wird durch eine mechanische Spannung das Material gedehnt und wechselt ab einer bestimmten Dehnung, aus energetischen Gründen (freie Gibbs'sche Energie), das Kristallsystem. Bei Wegnahme der äußeren Kraft kehrt das Material wieder in seine Ausgangsform und das ursprüngliche Kristallsystem zurück (es "erinnert" sich an seine ursprüngliche Form).

Neben der durch die Dehnung verursachten Widerstandsänderung (Längung bei gleichzeitiger Verjüngung) des Materials kommt somit noch eine Widerstandänderung durch den Wechsel des Kristallsystems hinzu. Im Rahmen der vorliegenden Idee wird die durch die Dehnung verursachte Änderung des ohmschen Widerstands als Sensorgröße genutzt. Da das System 1 am Körper getragen wird und sowohl die Körpertemperatur als auch die Umgebungstemperatur sich nicht wesentlich ändert, basiert die Widerstandsänderung im Wesentlichen auf der Dehnung des Formgedächtniswerkstoffes. Zur genaueren Bestimmung der Dehnung mittels des elektrischen Widerstandes kann zusätzlich die Temperatur gemessen werden, da sich der Widerstand eines ohmschen Leiters in Abhängigkeit der Temperatur ändert und zugleich einen Einfluss auf das vorliegende Kristallsystem hat.

Formgedächtnislegierungen basieren zumeist aus Nickel-Titan (NiTi) Basislegierungen, die in Abhängigkeit der geforderten Spezifikationen durch z.B. weitere Legierungselemente ergänzt oder einer thermo-mechanischen Vorbehandlung unterzogen werden.

Der Faden oder die strukturierte Flachformstruktur wird an die mechanischen Spezifikationen (Stellweg, Kraft) angepasst. Im Falle des Fadens wird dessen Dicke (130 µm, bevorzugt 127 µm, insbesondere kleiner 100 µm, insbesondere kleiner 70 ) und/oder Integration (geradlinig, mäanderförmig, im Zickzack, spiralförmig, u.a.) entsprechend gewählt. Im Falle der Flachformstruktur wird dessen geometrische Ausgestaltung (geradlinig, mäanderförmig, im Zickzack, spiralförmig, u.a.) angepasst.

Der Faden wird in den ersten Dehnungsbereich 3 eingewebt, gestrickt, gewirkt oder getuftet. Der Faden kann mäanderförmig in den ersten Dehnungsbereich 3 eingearbeitet sein.

Bei einer Relativbewegung eines ersten Körperteils 4a zu einem zweiten Körperteil 4b ergibt sich eine, insbesondere positive, Dehnung des ersten Dehnungsbereichs 3 mit dem Formgedächtniswerkstoff. Bei dem ersten und zweiten Körperteil 4a, 4b handelt es sich um eine Extremität, Gelenk, Wirbel, Muskel oder einen Knochen. Die Bewegung kann auch über mehrere Gelenke oder Wirbel hinweg verlaufen, etwa bei der Brustwirbelsäule.

Beispielsweise handelt es sich bei dem ersten Körperteil 4a um das Schulterblatt verbunden mit dem Schlüsselbein und beim zweiten Körperteil 4b um den Brustkorb, beispielsweise den hinteren Teil des Brustkorbs. Weitere Beispiele für das erste und zweite Körperteil 4a, 4b sind:
- oberer Wirbelkörper gegen tieferliegenden Wirbelkörper der Lendenwirbelsäule:
- oberer Wirbelkörper gegen tieferliegenden Wirbelkörper der Brustwirbelsäule;
- Brustwirbelsäule gegen Lendenwirbelsäule;
- am Fuß: der Rückfuß, der nach Innen abknickt und somit der inneren Anteil der Malleolengabel;
- am Fuß: der Rückfuß von der äußeren Malleolengabel beim Abknicken nach Außen;
- am Fuß: der Mittel-Vorfuß, der sich vom Unterschenkel entfernt, bei einer pathologischen Fußheberschwäche;
- an der Hüfte: Becken und Oberschenkel beim pathologischen Einknicken des Hüftgelenkes;
- am Knie: der mittlere Teil des Oberschenkels und Unterschenkels bei einem pathologischen Wegknicken nach Innen.

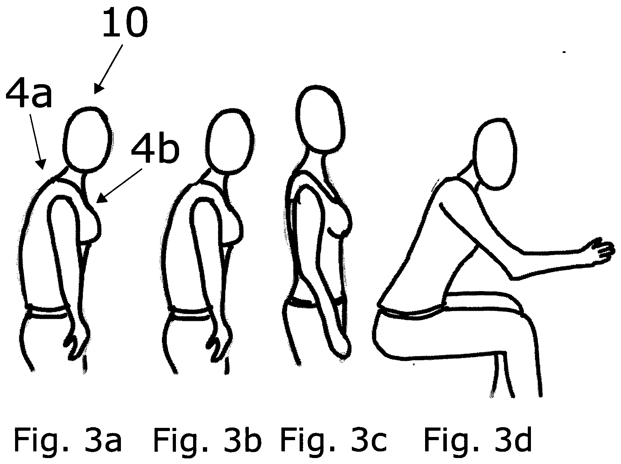

Fig. 3a-d zeigen verschiedene Bewegungsmuster, wobei nur die Fig. 3a mit Bezugszeichen versehen ist. Fig. 3a-c zeigen das Aufrichten von einer eher ungesunden Körperhaltung in Fig. 3a zu einer eher gesunden Körperhaltung in Fig. 3c. Fig. 3d zeigt ein "gesundes" Hinsetzen mit geradem Rücken. Es entsteht bei der Bewegung aus den Fig. 3a-c oder Fig. 3d eine Relativbewegung vom Schulterblatt ("erstes Körperteil 4a") zum Brustkorb ("zweites Körperteil 4b"). Bei dieser Bewegung dehnt bzw. entspannt sich der Dehnungsbereich 3.

Neben dem ersten Dehnungsbereich 3, der sich, wie oben erwähnt, bei entsprechender Bewegung positiv dehnt, umfasst das System einen zweiten Dehnungsbereich 13, der weicher ist als der erste Dehnungsbereich und sich beim Anziehen des Systems dehnt. Dieser Bereich 13 sorgt für Tragekomfort beim Anwender. Es können somit auch kleinere und größere Anwender das System 1 verwenden, da sich der zweite Bereich 13 entsprechend leicht dehnt. Dieser Bereich 14 ist etwa ein Gummizug wie man es von Textilien wie Schlafanzüge oder Unterwäsche kennt. Dadurch kann ebenso eine gewisse Vorspannung des System erreicht werden.

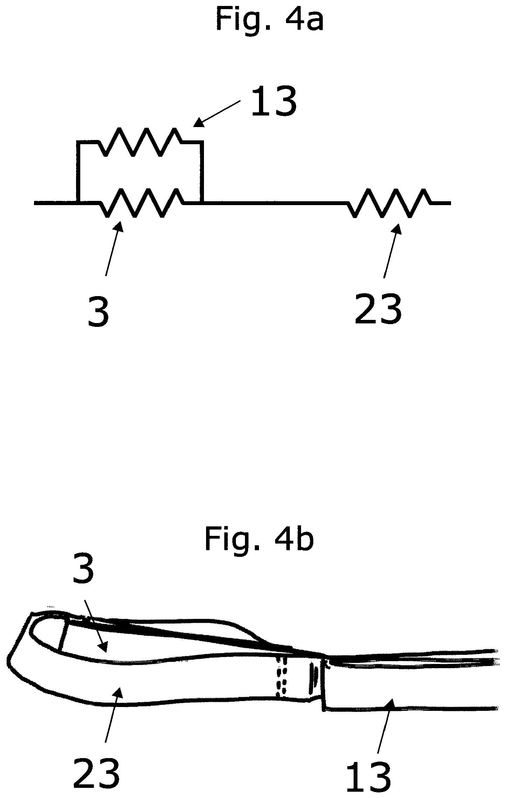

Das System 1 umfasst einen dritten Dehnungsbereich 23, der härter ist als der erste Dehnungsbereich 3 und eine Überlast des ersten Dehnungsbereichs 3 verhindert. So ist der dritte Dehnungsbereich 23 etwa um den ersten Dehnungsbereich 1 herum angeordnet, d.h. sowohl erster als auch dritter Dehnungsbereich 3, 23 greifen am ersten und zweiten Ende 3a, 3b an. Erreicht der erste Dehnungsbereich 3 seine Dehnungsgrenze, verhindert der dritte Dehnungsbereich 23, dass der erste Dehnungsbereich 3 weiter gedehnt wird. Diese sind "parallelgeschaltet". Fig. 4a zeigt den (mechanischen) Schaltplan. Fig. 4b zeigt diese Ausgestaltung. Der zweite Dehnungsbereich 13 ist dazu "in Serie" geschaltet. Entsprechend angeordnete andere Ausgestaltungen um ein Überdehnen zu verhindern sind möglich.

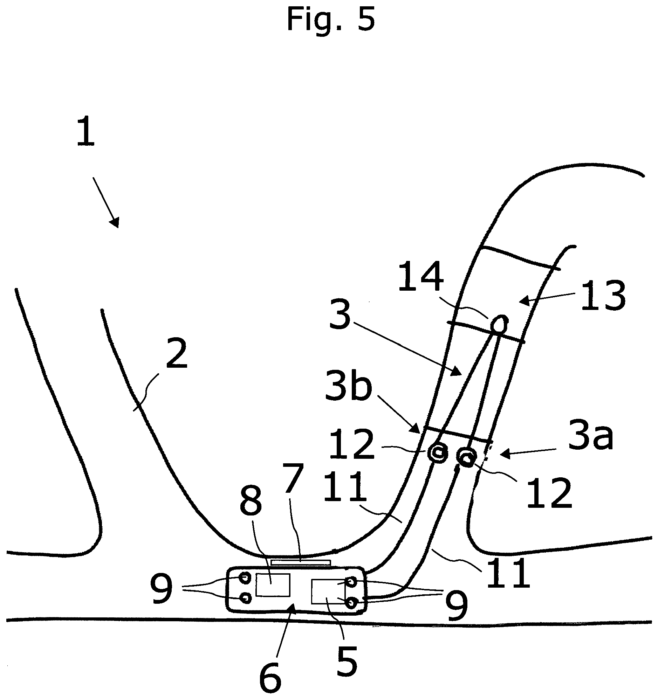

Fig. 5 zeigt einen Ausschnitt der Trägereinrichtung 2 mit dem Dehnungsbereich 3 und 13. Dargestellt ist die Ausgestaltung mit einem Faden aus einem Formgedächtniswerkstoff. Der Faden 3 ist durch entsprechende Halterungen 12 am ersten und zweiten Ende 3a, 3b befestigt, sowohl mechanisch als auch elektrisch. Über Leitungen 11 ist der Faden 3 an den Enden 3a, 3b mit einer Datenverarbeitungseinheit 6 verbunden. Der Faden 3 wird im Beispiel einmalig "umgelenkt", siehe Bezugszeichen 14. Mehrmaliges Umlenken ist ebenso möglich, dadurch verlängert sich der Weg und das Signal (siehe unten) wird entsprechend größer. Ebenso ist eine Ausgestaltung ohne Umlenkung möglich, das Signal wird entsprechend kleiner. Die Umlenkung kann als Gleitlager oder einfache Befestigung ausgestaltet sein, also als Gegenlager.

Fig. 5 zeigt das System 1 mit dem Dehnungsbereich 3 auf der rechten Seite des vertikalen Teils der Trägereinrichtung 2 (also das Schulterteil). Ohne erfinderische Zutun ist auch eine Ausgestaltung auf der linken Seite des vertikalen Teils der Trägereinrichtung 2 möglich. In einer Ausgestaltung umfassen beide Seiten einen Dehnungsbereich. In einer Ausgestaltung ist der Dehnungsbereich 3 in einem horizontalen Teil angeordnet.

Die Datenverarbeitungseinheit 6 muss nicht zwangsweise im horizontalen Teil untergebracht sein. Ausgestaltungen im linken oder rechten vertikalen Teil sind möglich.

Bewegt sich der Anwender wie etwa in den Fig. 3a-d gezeigt, dehnt sich der Faden 3 und der Widerstand ändert sich. Die Dehnung beträgt beispielsweise 100 µm oder bis zu einigen mm oder gar cm. Wie erwähnt, ist die Temperatur im Wesentlichen konstant.

Das System 1 umfasst zumindest einen Sensor 5, der mit dem ersten und zweiten Ende 3a, 3b verbunden ist. Der Sensor misst die Dehnung des Fadens 3 und sendet damit korrespondierende Signale an eine Datenverarbeitungseinheit 6. Der Sensor 5 ist also zur Messung des Widerstands ausgestaltet.

Beispielsweise umfasst dieser etwa eine Stromquelle, insbesondere eine Konstantstromquelle, und eine Spannungsmesseinheit. Es wird also ein konstanter Strom durch den Faden 3 geschickt, der Spannungsabfall gemessen und daraus der Widerstand durch die Datenverarbeitungseinheit 7 berechnet. In einer Ausgestaltung wird auf einen konstanten Strom geregelt und die Regelparameter sind korrespondierend zum Widerstandswert. Es können auch dedizierte Widerstandsmessungs-ASICs oder andere geeignete Bauteile zum Einsatz kommen.

Das System 1 umfasst auch die Datenverarbeitungseinheit 6, die mit dem Sensor 5 verbunden ist. Der Sensor 5 kann die Datenverarbeitungseinheit 6 integriert sein oder diskret vorliegen und mit der Datenverarbeitungseinheit 6 verbunden sein.

Das System 1 umfasst zumindest eine Feedbackeinheit 7, die mit der Datenverarbeitungseinheit 6 verbunden ist, wobei die Datenverarbeitungseinheit 6 dazu ausgestaltet ist, ein Signal an die Feedbackeinheit 6 zu senden, wenn die Dehnung größer als ein Schwellenwert ist. Die Feedbackeinheit 7 gibt einen taktilen Reiz an den Körper 10. Dadurch ergibt sich unmittelbar eine Rückmeldung an den Körper 10 und damit an den Anwender, wenn dieser eine ungesunde Haltung einnimmt. Die Feedbackeinheit 7 ist etwa ein Unwuchtmotor oder Vibrationsmotor.

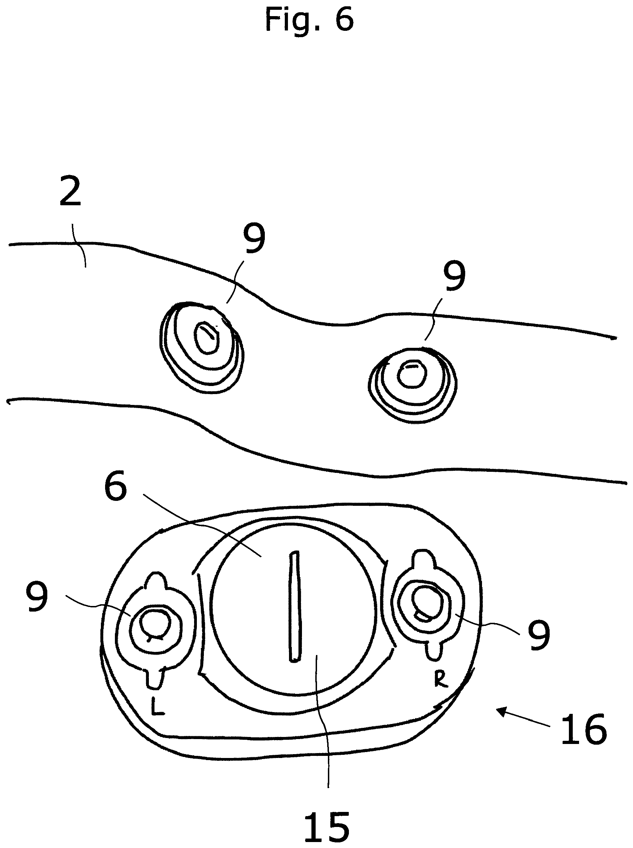

Fig. 6 zeigt eine Ausgestaltung für ein Gehäuse 16 samt Energieversorgung. Im oberen Bereich ist ein Abschnitt der Trägereinrichtung 2 zu sehen. Dieser umfasst erste Teile eines Druckknopfsystems 9. Siehe auch Fig. 5. Im unteren Teil von Fig. 6 ist eine Batterie bzw. Batteriehalterung 15 dargestellt. Diese umfasst zu den ersten Teilen korrespondierende zweite Teile eines Druckknopfsystems. Dadurch kann das Gehäuse 6 inklusive Batterie 15 von der Trägereinrichtung 2 gelöst werden und die Trägereinrichtung 2 kann dann gewaschen werden. Aus dem gleichen Zweck sind auch die Datenverarbeitungseinheit 6, Sensor 5, Feedbackeinheit 7 und Bluetooth-Chip 8 (siehe unten), im abnehmbaren Teil angeordnet (lediglich Datenverarbeitungseinheit 6 ist in Fig. 6 eingezeichnet).

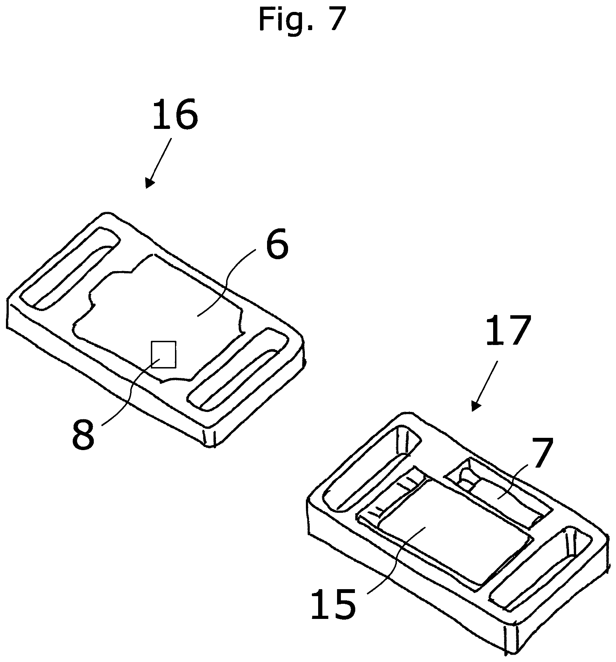

Fig. 7 zeigt eine Ausgestaltung für ein Gehäuse. Dieses besteht aus zwei Gehäusehälften 16 und 17, die entweder ineinander oder nebeneinander angeordnet werden können. In den Gehäusehälften kann die Batterie/Akku 5, die Datenverarbeitungseinheit 6 samt Bluetooth-Chip 8 und Feedbackeinheit 7 angeordnet sein. Die Hälften 16, 17 können auch ineinandergeschoben werden. Gegebenenfalls werden sie miteinander fixiert, etwa mittels Schrauben.

Ebenso ist eine Ausgestaltung mit nur einem Gehäuse möglich. Die o.g. Bauteile können dabei seitlich eingeschoben werden.

Das System 1 umfasst einen Bluetooth-Chip 8, der mit der Datenverarbeitungseinheit 6 verbunden ist, und dazu ausgestaltet ist eine Verbindung mit einem Mobilgerät aufzubauen. Der Bluetooth-Chip 8 kann in die Datenverarbeitungseinheit 6 integriert sein oder diskret vorliegen und mit der Datenverarbeitungseinheit 6 verbunden sein. Der Bluetooth-Chip 8 ist Bluetooth 4.0 geeignet, insbesondere mit dem Protokollstapel Bluetooth Low Energy. Alternativ kann als Funkmodul auch eine andere Funktechnologie verwendet werden, etwa WLAN (aus der IEEE-802.11-Familie), ZigBee, ANT, ANT+, Long Range Wide Area Network (LoRaWAN), GSM, GPRS, EDGE, LTE, 5G oder weitere Funkstandards. Auch ist eine Kombination von mehreren Funkstandards auf einem Modul möglich, etwa ZigBee und Bluetooth. Ebenso ist eine drahtgebundene Verbindung möglich, etwa über USB.

Auf dem Mobilgerät, etwa ein Smartphone, Tablet, Laptop, PC oder auch stationärer Computer, läuft eine Software/App. Über die App kann die Datenverarbeitungseinheit 6, der Sensor 5 und die Feedbackeinheit 7 konfiguriert, ausgelesen und gesteuert werden. So kann etwa ein Grundzustand eingestellt werden oder eine Kalibrierung durchgeführt werden.

Mit dieser App können also etwa verschiedene Profile angelegt werden, wann der taktile Reiz über die Feedbackeinheit 7 ausgelöst werden soll. Dies führt zu einer aktiven Haltungskorrektur und (beispielsweise) Schulterblattaufrichtung mittels taktilem Reiz (Biofeedback) über die Aufrichtung der Schulter und daraus resultierend der Wirbelsäule, bei protrahierten Schultern (also, wenn die Schultern nach vorne fallen).

Es können verschiedene Aktivitätsprofile/Zeitprofile über die App eingestellt werden. Beispiele für Aktivitäts- oder Zeitprofile sind:
- Normaler Tag, hier wird der taktile Reiz erst ausgelöst, wenn die Schultern weniger als 5 Min. pro 10 Min. in Retraktion (aufgerichtet) stehen.
- Haushaltsprofil, hier wird der taktile Reiz erst ausgelöst, wenn über einen längeren Zeitraum als 10 Min. die Schultern in Protraktion (nach vorne geneigt) stehen.
- PC-Arbeitsprofil, hier wird der taktile Reiz erst ausgelöst, wenn die Schultern weniger als 30 Min pro Stunde in Retraktion (aufgerichtet) stehen.
- Sportprofil, je nach Sportart, z.B. beim Reiten: hier wird der taktile Reiz erst ausgelöst, wenn die Schultern weniger als 3 Min. pro 5 Min. in Retraktion (aufgerichtet) stehen; beim Kraftsport wird kontinuierlich darauf geachtet, dass mit geradem Rücken trainiert wird.

Ebenso können Intensitätsprofile der Schulteraufrichtung angelegt werden. Es wird also über die App gesteuert, ab welcher Dehnung der taktile Reiz ausgelöst wird. Hierdurch kann die Aufrichtung dosiert werden, das heißt eine leichte Protraktion (nach vorne geneigt) der Schulter löst noch keinen Reiz aus. So kann man das Biofeedback an die individuellen Bedürfnisse und Möglichkeiten des Trägers anpassen.

Die Funkverbindung zwischen dem System 1 und der App ist nur notwendig um Messwerte auszulesen und Einstellungen zu verändern. Das System ist auch ohne diese Verbindung völlig autark nutzbar und agiert dann innerhalb der zuletzt eingestellten Parameter und Profile.

In einer Ausgestaltung sind zwei unterschiedliche Apps vorgesehen: Eine App zum initialen Anpassen an den Träger durch Anpassung der Sensorgrenzen und Intensitätswerte, welche von einem Physiotherapeuten oder versiertem Anwender verwendet wird. Vom eigentlichen Träger wird eine andere App (oder die gleiche App mit reduziertem Funktionsumfang) verwendet, um im Laufe des Tages zwischen verschiedenen Aktivitätsprofilen zu wechseln, beispielsweise Büroarbeit, Sport usw.

Außerdem kann eine App aktuelle und gespeicherte Daten (Messwerte und Ereignisse) aus der Datenverarbeitungseinheit auslesen, so dass das Beugeverhalten des Trägers analysiert und die Häufigkeit der ausgelösten Biofeedbacks erfasst werden kann. Diese Informationen sind für die Verbesserung der Therapie sehr hilfreich und können auch dem Träger als Motivation dienen.

### Bezugszeichenliste

- 1: System
- 2: Trägereinrichtung
- 3: erster Dehnungsbereich
- 3a: erstes Ende
- 3b: zweites Ende
- 4a: erstes Körperteil
- 4b: zweites Körperteil
- 5: Sensor
- 6: Datenverarbeitungseinheit
- 7: Feedbackeinheit
- 8: Bluetooth-Chip
- 9: Druckknopf
- 10: Körper
- 11: Verbindungsleitung
- 12: Befestigung
- 13: zweiter Dehnungsbereich
- 14: Umlenkung
- 15: Batterie
- 16: Gehäusehälfte
- 17: Gehäusehälfte
- 23: dritter Dehnungsbereich

## Patentansprüche

1. System (1) zur Korrektur einer Körperhaltung, umfassend
- eine Trägereinrichtung (2) zur Anbringung am menschlichen Körper (10) mit zumindest einem ersten Dehnungsbereich (3),
wobei der erste Dehnungsbereich (3) einen Formgedächtniswerkstoff mit einem ersten Ende (3a) und einem zweiten Ende (3b) beinhaltet, insbesondere zumindest einen Faden aus einem Formgedächtniswerkstoff oder eine Flachformstruktur beinhaltet, und
wobei sich bei einer Relativbewegung eines ersten Körperteils (4a) zu einem zweiten Körperteil (4b) eine, insbesondere positive, Dehnung des ersten Dehnungsbereichs (3) mit dem Formgedächtniswerkstoff ergibt, wobei es sich bei dem ersten und zweiten Körperteil (4a, 4b) um eine Extremität, Gelenk, Wirbel, Muskel oder einen Knochen handelt;
- zumindest einen Sensor (5), der mit dem ersten und zweiten Ende (3a, 3b) verbunden ist, und dazu ausgestaltet ist, eine Dehnung zu detektieren und damit korrespondierende Signale an eine Datenverarbeitungseinheit (6) zu senden;
- die Datenverarbeitungseinheit (6), die mit dem Sensor (5) verbunden ist; und
- zumindest eine Feedbackeinheit (7), die mit der Datenverarbeitungseinheit (6) verbunden ist,
wobei die Datenverarbeitungseinheit (6) dazu ausgestaltet ist, ein Signal an die Feedbackeinheit (7) zu senden, wenn die Dehnung größer als ein Schwellenwert ist und diese eine Rückmeldung an den Körper (10) abgibt.

2. System (1) nach Anspruch 1,
wobei der Sensor (5) zur Messung des Widerstands ausgestaltet ist, insbesondere umfasst der Sensor (5) eine Stromquelle, insbesondere eine Konstantstromquelle, und eine Spannungsmesseinheit.

3. System (1) nach Anspruch 1 oder 2,
wobei der Formgedächtniswerkstoff, insbesondere der zumindest eine Faden oder die eine Flachformstruktur, in den ersten Dehnungsbereich (3) eingewebt, gestrickt, gewirkt oder getuftet ist.

4. System (1) nach zumindest einem der vorherigen Ansprüche,
wobei es sich bei dem Formgedächtniswerkstoff um eine Nickel-Titan-Basislegierung handelt.

5. System (1) nach zumindest einem der vorherigen Ansprüche, weiter umfassend
- einen Bluetooth-Chip (8), der mit der Datenverarbeitungseinheit (6) verbunden ist, und dazu ausgestaltet ist eine Verbindung mit einem Mobilgerät aufzubauen.

6. System (1) nach zumindest einem der vorherigen Ansprüche,
wobei das System einen zweiten Dehnungsbereich (13) umfasst, der weicher ist als der erste Dehnungsbereich (3) und sich beim Anziehen des Systems (1) dehnt.

7. System (1) nach zumindest einem der vorherigen Ansprüche,
wobei das System einen dritten Dehnungsbereich (23) umfasst, der härter ist als der erste Dehnungsbereich (3) und eine Überlast des ersten Dehnungsbereichs (3) verhindert.

8. System (1) nach zumindest einem der vorherigen Ansprüche,
wobei es sich bei dem ersten Körperteil (4a) um das Schulterblatt verbunden mit dem Schlüsselbein und beim zweiten Körperteil (4b) um den Brustkorb, insbesondere den hinteren Teil des Brustkorbs, handelt.

## Claims

1. System (1) for correcting body posture, comprising
- a carrier device (2) to be attached to the human body (10) with at least one first stretchable region (3),
wherein the first stretchable region (3) comprises a shape memory material having a first end (3a) and a second end (3b), in particular comprising at least one thread made of a shape memory material or a flat shape structure of a shape memory material, and
wherein, upon relative movement of a first body part (4a) with respect to a second body part (4b), a, in particular positive, elongation of the first stretchable region (3) comprising the shape memory material occurs, wherein the first and second body part (4a, 4b) are a limb, a joint, a vertebra, a muscle or a bone;
- at least one sensor (5) connected to the first and second ends (3a, 3b the sensor being configured to detect an elongation and to transmit corresponding signals to a data processing unit (6);
- the data processing unit (6) connected to the sensor (5); and
- at least one feedback unit (7) connected to the data processing unit (6), wherein the data processing unit (6) is designed to transmit a signal to the feedback unit (7) when the strain is greater than a threshold value and the feedback unit (7) then provides feedback to the body (10).

2. System (1) according to claim 1,
wherein the sensor (5) is configured to measure resistance, in particular comprising a current source, preferably a constant current source, and a voltage measuring unit.

3. System (1) according to claim 1 or 2,
wherein the shape memory material, in particular at least one thread or flat shape structure, is woven, knitted, crocheted or tufted into the first stretch area (3).

4. System (1) according to at least one of the previous claims,
wherein the shape memory material is a nickel-titanium-based alloy.

5. System (1) according to any of the previous claims, further comprising
- a Bluetooth chip (8) which is connected to the data processing unit (6) and is designed to establish a connection with a mobile device.

6. System (1) according to any of the previous claims,
wherein the system comprises a second strain region (13) that is softer than the first strain region (3) and stretches when the system (1) is tightened.

7. System (1) according to any of the previous claims,
wherein the system comprises a third stretch area (23) that is harder than the first stretch area (3) and prevents overloading of the first stretch area (3).

8. The system (1) according to any of the preceding claims,
wherein the first body part (4a) comprises the scapula connected to the clavicle and the second body part (4b) comprises the thorax, in particular a posterior portion of the thorax.

## Revendications

1. Système (1) de correction d'une posture corporelle, comprenant :
un dispositif porteur (2) destiné à être fixé sur le corps humain (10), comprenant au moins une première zone extensible (3) ;
ladite première zone extensible (3) comprenant un matériau à mémoire de forme ayant une première extrémité (3a) et une deuxième extrémité (3b), comprenant en particulier au moins un fil en matériau à mémoire de forme ou une structure plane en matériau à mémoire de forme ; et
lors d'un mouvement relatif d'une première partie du corps (4a) par rapport à une deuxième partie du corps (4b),produisant un allongement en particulier positive, de la première zone extensible (3) comprenant le matériau à mémoire de forme, la première et la deuxième partie du corps (4a, 4b) étant constituées d'un membre, d'une articulation, d'une vertèbre, d'un muscle ou d'un os ;
au moins un capteur (5) relié aux première et deuxième extrémités (3a, 3b), ledit capteur étant conçu pour détecter ledit allongement et transmettre des signaux correspondants à une unité de traitement de données (6) ;
l'unité de traitement de données (6) reliée au capteur (5) ; et
au moins une unité de rétroaction (7) reliée à l'unité de traitement de données (6), ladite unité de traitement de données (6) étant conçue pour transmettre un signal à l'unité de rétroaction (7) lorsque l'allongement dépasse une valeur seuil, et ladite unité de rétroaction (7) fournissant une rétroaction au corps (10).

2. Système (1) selon la revendication 1,
dans lequel le capteur (5) est conçu pour mesurer une résistance, comprenant en particulier une source de courant, de préférence une source de courant constant, et une unité de mesure de tension.

3. Système (1) selon l'une quelconque des revendications précédentes,
dans lequel le matériau à mémoire de forme, en particulier ledit au moins un fil ou ladite structure plane, est tissé, tricoté, trame ou tufté dans la première zone extensible (3).

4. Système (1) selon l'une quelconque des revendications précédentes,
dans lequel le matériau à mémoire de forme comprend un alliage à base de nickel-titane.

5. Système (1) selon l'une quelconque des revendications précédentes, comprenant en outre :
une puce Bluetooth (8) reliée à l'unité de traitement de données (6), ladite puce Bluetooth étant conçue pour établir une connexion avec un dispositif mobile.

6. Système (1) selon l'une quelconque des revendications précédentes,
dans lequel le système (1) comprend en outre une deuxième zone extensible (13) plus souple que la première zone extensible (3) et s'étendant lors de la mise en place du système (1).

7. Système (1) selon l'une quelconque des revendications précédentes,
dans lequel le système (1) comprend en outre une troisième zone extensible (23) plus rigide que la première zone extensible (3) et empêchant une surcharge de la première zone extensible (3).

8. Système (1) selon l'une quelconque des revendications précédentes,
dans lequel la première partie du corps (4a) comprend l'omoplate reliée à la clavicule et la deuxième partie du corps (4b) comprend le thorax, en particulier une partie postérieure du thorax.
